(19) Europäisches Patentamt
European Patent Office
Office européen des brevets

(11) Veröffentlichungsnummer: **0 083 428**
**B1**

(12) **EUROPÄISCHE PATENTSCHRIFT**

(45) Veröffentlichungstag der Patentschrift: **10.07.85**

(51) Int. Cl.⁴: **C 07 C 37/14**, C 07 C 39/14

(21) Anmeldenummer: **82111521.9**

(22) Anmeldetag: **11.12.82**

# (54) Verfahren zur Herstellung von in 1-Stellung unsubstituierten, durch araliphatische Reste substituierten beta-Naphtholen.

(30) Priorität: **31.12.81 DE 3152004**

(43) Veröffentlichungstag der Anmeldung: **13.07.83 Patentblatt 83/28**

(45) Bekanntmachung des Hinweises auf die Patenterteilung: **10.07.85 Patentblatt 85/28**

(84) Benannte Vertragsstaaten: **BE CH DE FR GB LI**

(56) Entgegenhaltungen:
**EP - A - 0 012 297**
**EP - A - 0 030 701**
**US - A - 2 247 403**
**US - A - 2 523 939**
**US - A - 2 714 120**
**US - A - 2 978 515**
**US - A - 3 267 154**

**JOURNAL OF ORGANIC CHEMISTRY, Band 17, 1952, Seiten 243-248, Easton, USA**

(73) Patentinhaber: **BASF Aktiengesellschaft, Carl-Bosch-Strasse 38, D-6700 Ludwigshafen (DE)**

(72) Erfinder: **Kast, Hellmut, Dr., An der Tuchbleiche 4, D-6712 Bobenheim-Roxheim (DE)**
Erfinder: **Merger, Franz, Dr., Max-Slevogt-Strasse 25, D-6710 Frankenthal (DE)**
Erfinder: **Nestler, Gerhard, Dr., Van-Leyden-Strasse 17, D-6700 Ludwigshafen (DE)**
Erfinder: **Zeidler, Georg, Mutterstadter Strasse 7, D-6701 Dannstadt-Schauernheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die Erfindung betrifft ein Verfahren zur Herstellung von in 1-Stellung unsubstituierten, durch araliphatische Reste substituierten β-Naphtholen durch Umsetzung von β-Naphtholen mit Styrolen in Gegenwart von unter den Reaktionsbedingungen inerten, organischen Lösungsmitteln mit einem Siedepunkt über 130°C und in Gegenwart von aromatischen oder aliphatischen Sulfonsäuren.

Es ist bekannt, daß β-Naphthole in Gegenwart von Alkylierungskatalysatoren, z. B. $H_2SO_4$, $H_3PO_4$, $BF_3$ oder $AlCl_3$ (Houben—Weyl, Methoden der Organischen Chemie, Band 6/1c, II, Seiten 955 bis 956) mit Styrolen umgesetzt werden können. Da es jedoch unter den Alkylierungsbedingungen zur teilweisen Polymerisation der Styrole (US-PS 2 714 120, Spalte 1, Zeilen 28—32), zur Bildung von Dinaphthylethern (Houben—Weyl, loc. cit., Band VI/3, Seite 85) und bei Verwendung von $H_2SO_4$ als Katalysator zur Bildung von Naphtholsulfonsäuren (Houben—Weyl, loc. cit., Band 9, Seite 487) kommt, sind die Ausbeuten allgemein unbefriedigend (s. Vergleichsbeispiel 2 und 3).

Die US-PS 2 714 120 beschreibt ein Alkylierungsverfahren, das in Gegenwart von verdünnter Schwefelsäure bzw. Aryl- oder Alkylsulfonsäuren durchgeführt wird. Eine Polymerisation des Styrols tritt in geringerem Maße auf. Die Umsetzungen erfolgen bei 50—300°C in Gegenwart eines niedrigsiedenden Lösungsmittels oder lösungsmittelfrei (Spalte 1, Zeilen 59—66). Wie Beispiele 9 und 13 der Patentschrift zeigen, werden bei der Umsetzung von β-Naphthol mit Styrol bzw. α-Methylstyrol in Gegenwart von aromatischen oder aliphatischen Sulfonsäuren als Katalysatoren keine Lösungsmittel verwendet; die Endstoffe sind hauptsächlich in 1-Stellung aralkylierte β-Naphthole (Spalte 6, Zeilen 17—64).

Die GB-PS 1 111 751 beschreibt die Aralkylierung von Naphtholen mit Styrol in Gegenwart von Oxalsäure. Beispiele für derartige Umsetzungen werden jedoch in der Patentschrift nicht angegeben. Beispiel 5 (Vergleichsbeispiel) zeigt, daß in Gegenwart von Oxalsäure hauptsächlich 1-(α-Phenylethyl)-naphthol-2 gebildet wird.

DE-OS 2 950 290 erwähnt, daß in der Regel die Alkylierung von 2-Naphthol mit Olefinen oder Alkoholen in Gegenwart von anorganischen Säuren oder organischen Sulfonsäuren vor allem zu 6-alkylierten 2-Naphtholen führt. In J. Org. Chem., Band 17 (1952), Seite 247, wird zwar die Herstellung von 6-(α-Phenyläthyl)-2-naphthol durch Umsetzung von 2-Naphthol mit Styrol beschrieben, doch Angaben bezüglich der Ausbeute fehlen. Die angegebenen »92%« beziehen sich lediglich auf das Monoalkylierungsprodukt ((α-Phenylethyl)-2-naphthole). Führt man eine entsprechende Vergleichsumsetzung durch, so erhält man 6-(α-Phenylethyl)-2-naphthol mit einer Ausbeute von lediglich 34% der Theorie (s. Beispiel 5). Bei allen diesen Verfahren bilden sich wesentliche Mengen von Dinaphthylethern und Styrololigomeren. Die DE-OS 29 50 290 zeigt ein Verfahren zur Herstellung von 1-(Aralkyl)-2-naphtholen durch Umsetzung von 2-Naphtholen mit Styrolen in Gegenwart einer Carbonsäure. Vergleichsbeispiel 2 zeigt, daß die Umsetzung mit Schwefelsäure als Katalysator bei 120°C in Abwesenheit von Lösungsmitteln nur zu unbefriedigenden Mengen an 6-(α-Phenylethyl)-2-naphthol (21%), die außerdem noch weitere 21% der in 3-Stellung substituierten Verbindung enthalten, führt. Insbesondere tritt der störende Dinaphthylether in relativ größerer Menge (6%) auf. Ebenso sind bei allen diesen Schwefelsäureumsetzungen stets gewisse Mengen an Naphtholsulfonsäuren zu beobachten.

Vergleichsbeispiel 3 zeigt, wie empfindlich die Umsetzung auf jede Veränderung der Komponenten reagiert, denn mit Phosphorsäure als Katalysator tritt wieder ein Anteil an 1-Verbindung, der weit höher als der 6-Anteil ist, als Endstoff auf.

In der GB-PS 731 270 wird die Alkylierung von Phenolen und Naphtholen in Gegenwart von Kationenaustauschern beschrieben. Bezüglich einer Umsetzung von β-Naphthol mit Styrol finden sich jedoch weder im allgemeinen Teil noch im experimentellen Teil nähere Angaben. Führt man eine entsprechende Umsetzung in Gegenwart von ®Amberlite IR 120 (siehe Beispiel 1 der GB-PS 731 270) durch, so erhält man ein Gemisch aus 14% β-Naphthol, 63% 1-(1′-Phenylethyl)-2-naphthol, 10% 6-(1′-Phenylethyl)-2-naphthol, 3% 3- und 8-(1′-Phenylethylnaphthol) und 9% zweifach und höher alkylierte Naphthole (siehe Beispiel 6).

Insgesamt liefern alle diese Verfahren keine β-Naphthole, die keine deutlichen Mengen an 1-substituierten, andererseits aber höhere Mengen an 6-substituierten Naphtholen, die wesentlich über den Anteilen an 3- oder 8-substituierten Naphtholen liegen, sowie als restliche Anteile 3,6-, 3,8-, 6,8- und 3,6,8-substituierte Naphthole enthalten und keine deutlichen Mengen an Dinaphthylether, Styrolpolymeren und Naphtholsulfonsäuren aufweisen.

Es wurde nun gefunden, daß man in 1-Stellung unsubstituierte, durch araliphatische Reste substituierte β-Naphthole der Formel

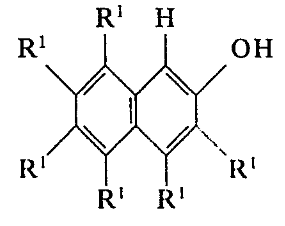

(I)

worin die einzelnen Reste $R^1$ gleich oder verschieden sein können, 1 bis 3 Reste $R^1$ jeweils den Rest

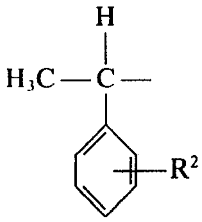

bedeuten, die übrigen Reste $R^1$ jeweils ein Wasserstoffatom oder einen Alkylrest mit 1 bis 8 Kohlenstoffatomen bezeichnen, $R^2$ für ein Wasserstoffatom oder einen Alkylrest mit 1 bis 8 Kohlenstoffatomen steht, durch Umsetzung von Naphtholen mit Styrolen in Gegenwart von aromatischen oder aliphatischen Sulfonsäuren vorteilhaft erhält, wenn man $\beta$-Naphthole der Formel

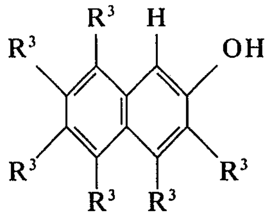

(II)

worin die einzelnen Reste $R^3$ gleich oder verschieden sein können und in den Stellungen, wo 1 bis 3 Reste $R^1$ den Rest

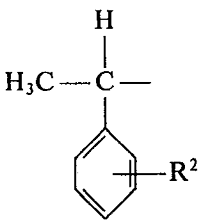

bedeuten, jeweils der entsprechende Rest $R^3$ ein Wasserstoffatom bezeichnet, die übrigen Reste $R^3$ dem Rest $R^1$ in der jeweiligen Stellung entsprechend ein Wasserstoffatom oder den Alkylrest von $R^1$ bedeuten, mit Styrolen der Formel

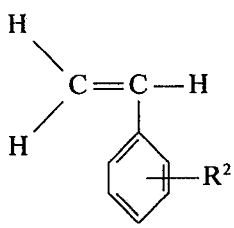

(III)

worin $R^2$ die vorgenannte Bedeutung besitzt, in Gegenwart von unter den Reaktionsbedingungen inerten, organischen Lösungsmitteln mit einem Siedepunkt über 130°C bei einer Temperatur von 100 bis 180°C umsetzt.

Die Umsetzung kann für den Fall der Verwendung von Styrol und 2-Naphthol durch die folgenden Formeln wiedergegeben werden:

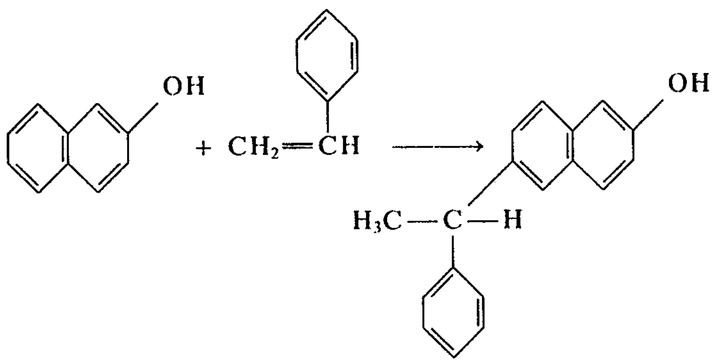

Im Vergleich zu den bekannten Verfahren liefert das Verfahren nach der Erfindung überraschend auf einfachem und wirtschaftlichem Wege 6-(Aralkyl)-2-naphthole sowie 3-, 8-, 3,6-, 6,8-, 3,8-, 3,6,8-substituierte 2-Naphthole in besserer Raum-Zeit-Ausbeute und Reinheit. Überraschend im Hinblick auf die deutsche Offenlegungsschrift, die US-Patentschrift und die britische Patentschrift ist ein Anteil an 1-substituiertem Endstoff nicht zu beobachten. Im Hinblick auf die Vergleichsbeispiele der deutschen Offenlegungsschrift war nicht zu erwarten, daß Nebenprodukte wie Dinaphthylether nicht beobachtet werden. Im Hinblick auf das Verfahren der US-Patentschrift, das organische Sulfonsäuren als Katalysatoren und keinen Lösungsmittelzusatz verwendet, ist es überraschend, daß gerade die Verwendung der erfindungsgemäßen Lösungsmittel zu hohen Mengen der erfindungsgemäßen Endstoffe ohne deutliche Bildung von 1-substituierten Naphtholen führt.

Die Zahl der eingeführten Aralkylgruppen III hängt stark vom Reaktandenverhältnis und vom Substitutionsgrad des eingesetzten Naphthols II ab. Die Aralkylierung erfolgt hauptsächlich in C-6-, C-3- und C-8-Stellung, vorzugsweise in C-6-Stellung. Die Ausgangsstoffe werden in stöchiometrischem Verhältnis oder im Überschuß, vorzugsweise in einem Verhältnis von 0,1 bis 5, vorteilhaft von 0,5 bis 2 Mol Ausgangsstoff III je Mol Naphthol II, vorteilhaft im Falle der Herstellung von in der Hauptsache einfach aralkylierten Naphtholen 0,1 bis 2, insbesondere 0,2 bis 0,5 Mol Ausgangsstoff III je Mol Ausgangsstoff II, im Falle von in großem Anteil zweifach aralkylierten Naphtholen 1,5 bis 3, insbesondere 2 bis 2,5 Mol Ausgangsstoff III je Mol Ausgangsstoff II, von in großem Anteil dreifach aralkylierten Naphtholen 2,5 bis 4, insbesondere 3 bis 3,5 Mol Ausgangsstoff III je Mol Ausgangsstoff II.

Bevorzugte Ausgangsstoffe II, III und dementsprechend bevorzugte Endstoffe I sind solche, in deren Formeln im Rahmen der im Patentanspruch angegebenen Definition die einzelnen Reste $R^1$ gleich oder verschieden sein können und 1 bis 3 Reste $R^1$ jeweils den Rest

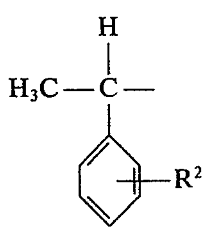

bedeuten, die übrigen Reste $R^1$ jeweils ein Wasserstoffatom oder einen Alkylrest mit 1 bis 4 Kohlenstoffatomen bedeuten, $R^2$ ein Wasserstoffatom oder einen Alkylrest mit 1 bis 4 Kohlenstoffatomen bezeichnet, die Reste $R^3$ gleich oder verschieden sein können und in den Stellungen, wo in der Formel I 1 bis 3 Reste $R^1$ den Rest

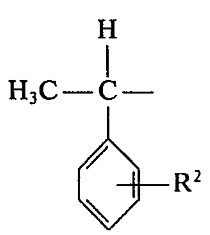

bedeuten, jeweils der entsprechende Rest $R^3$ ein Wasserstoffatom bezeichnet, die übrigen Reste $R^3$ dem Rest $R^1$ in der jeweiligen Stellung der Formel I entsprechend ein Wasserstoffatom oder den

Alkylrest von $R^1$ mit 1 bis 4 Kohlenstoffatomen bedeuten.

Folgende Naphthole kommen z. B. als Ausgangsstoffe II in Betracht: Methyl-, Ethyl-, n-Propyl-, Isopropyl-, n-Butyl-, Isobutyl-, sek.-Butyl-β-naphthol, wobei der Alkylsubstituent in 3-, 4-, 5-, 6-, 7- oder 8-Stellung sitzt; in 3,4-, 4,5-, 4,6-, 6,7-Stellung durch Methyl-, Ethyl-, n-Propyl-, Isopropyl-, n-Butyl-, Isobutyl-, sek.-Butyl-gruppen disubstituiertes β-Naphthol; entsprechende β-Naphthole mit 2 vorgenannten, aber unterschiedlichen Resten, z. B. 4-Ethyl-8-methyl-2-naphthol, 3-Methyl-4-butyl-2-naphthol; vorzugsweise β-Naphthol.

Es können z. B. folgende Styrole als Ausgangsstoffe III verwendet werden: Styrol; in o-, m- oder p-Stellung durch die Methyl-, Ethyl-, Propyl-, Isopropyl-, Butyl-gruppe substituierte Styrole.

Die Umsetzung wird bei einer Temperatur von 100 bis 180°C, vorzugsweise von 130 bis 150°C, mit Unterdruck, Überdruck oder drucklos, diskontinuierlich oder kontinuierlich durchgeführt. Man verwendet unter den Reaktionsbedingungen inerte Lösungsmittel mit einem Siedepunkt über 130°C, zweckmäßig von 131 bis 300°C, vorteilhaft 150 bis 250°C. Als Lösungsmittel kommen z. B. in Frage: Halogenkohlenwasserstoffe, insbesondere Fluor-, Brom- oder Chlorkohlenwasserstoffe, z. B. 1,2-Dibromethan, 1,1,2,2-Tetrachlorethan; aromatische Kohlenwasserstoffe, z. B. Naphthalin, Diphenyl, Dichlorbenzol, Chlorbenzol, Xylole, Ethylbenzol; aliphatische Kohlenwasserstoffe wie Dekalin, Testbenzin, Terpentinöl; und entsprechende Gemische. Bevorzugt sind Chlorbenzol, Dichlorbenzol, Dekalin, Testbenzin, die unter der Bezeichnung ®Shellsol F und Shellsol AB im Handel erhältlichen Lösungsmittelgemische aliphatischer bzw. aromatischer Kohlenwasserstoffe. Zweckmäßig verwendet man das Lösungsmittel in einer Menge von 10 bis 1000 Gewichtsprozent, vorzugsweise von 50 bis 200 Gewichtsprozent, bezogen auf Styrol III.

Als Sulfonsäuren kommen aromatische und aliphatische, vorteilhaft Alkylsulfonsäure mit 1 bis 8, insbesondere 1 bis 4 Kohlenstoffatomen, oder eine Alkylphenylsulfonsäure mit jeweils 7 bis 12 Kohlenstoffatomen oder Benzolsulfonsäure in Betracht. Die Sulfonsäure wird vorzugsweise in einem Verhältnis von 0,01 bis 1, insbesondere 0,05 bis 0,5 Äquivalenten Sulfonsäure je Mol Ausgangsstoff II, verwendet.

Beispielsweise sind folgende Sulfonsäuren geeignet: Benzol-, p-Toluol-, Ethylbenzol-, Propan-, Butan-, 2,5-Dimethylbenzol-, Methan-, Ethansulfonsäure.

Die Reaktion kann wie folgt durchgeführt werden: Ein Gemisch von Ausgangsstoff II und III, Sulfonsäure und Lösungsmittel wird während 1 bis 10 Stunden bei der Reaktionstemperatur gehalten. Zweckmäßig gibt man zu einem Gemisch von β-Naphthol II und Lösungsmittel bei der Reaktionstemperatur unter Rühren Styrol oder ein Gemisch aus Styrol und einem Teil des Lösungsmittels zu. Dann wird aus dem Reaktionsgemisch der Endstoff in üblicher Weise, z. B. durch Destillation, isoliert.

Die nach dem Verfahren der Erfindung herstellbaren (Aralkyl)-2-naphthole I sind wertvolle Ausgangsstoffe für die Herstellung von Farbstoffen, Schädlingsbekämpfungsmitteln, Pharmazeutika, Salben, Emulgatoren, Dispergiermitteln, Stabilisatoren, hydraulischen Flüssigkeiten, Weichmachern, Korrosionsinhibitoren, Desinfektionsmitteln, Pflanzenschutzmitteln. Sie finden beispielsweise bei der Herstellung von Farbstoffen zur Mineralölfärbung, Heizöl-, Wachs-, Fettfärbung Verwendung (DE-OS 2 852 863). Bezüglich der Verwendung wird auch auf die vorgenannten Veröffentlichungen verwiesen.

## Beispiel 1

In einem Rührreaktor wurde ein Gemisch aus 100 Gramm β-Naphthol und 100 Gramm ®Shellsol AB unter Stickstoff auf 130°C erhitzt, 3 Gramm p-Toluolsulfonsäuremonohydrat zugegeben und anschließend 100 Gramm Styrol innerhalb von 3 Stunden zugefügt. Nach Beendigung der Styrolzugabe wurde 3 Stunden bei 130°C gerührt. Entsprechend der gaschromatographischen Analyse enthielt das Reaktionsgemisch (196 Gramm) 3% β-Naphthol, 40% 6-(α-Phenylethyl)-β-naphthol, 7% eines Gemisches von 3- und 8-(α-Phenylethyl)-β-naphtholen, 39% eines Gemisches von 3,6-, 3,8- und 6,8-Di-(α-Penylethyl)-β-naphthole und 10% 3,6,8-Tri-(α-phenylethyl)-β-naphthol. Dinaphthylether und Styrololigomere traten nicht in deutlicher Menge auf.

## Beispiel 2

(Vergleichsbeispiel)

In einem Rührreaktor wurde unter Stickstoff ein Gemisch aus 100 Gramm β-Naphthol und 3 Gramm 96prozentiger Schwefelsäure bei 130°C innerhalb von 2 Stunden mit 100 Gramm Styrol versetzt und nach Beendigung der Styrolzugabe noch 2 Stunden bei 130°C gerührt. Entsprechend der gaschromatographischen Analyse enthielt das Reaktionsgemisch (205 Gramm) 2% β-Naphthol, 2% Dinaphthylether, 45% 1-, 6- und 3-(α-Phenylethyl)-β-naphthole, 34% Di-(α-phenylethyl)-β-naphthole und 6% Tri-(α-phenylethyl)-β-naphthole. Der Rest (11%) bestand aus Oligomeren des Styrols, höher aralkylierten Dinaphthylethern und aus Naphtholsulfonsäuren.

Beispiel 3

In einem Rührreaktor wurde ein Gemisch aus 100 Gramm β-Naphthol und 100 Gramm 1,2-Dichlorbenzol unter Stickstoff auf 135°C erhitzt, 2 Gramm p-Toluolsulfonsäuremonohydrat zugefügt und anschließend 50 Gramm Styrol innerhalb von 2 Stunden zugefügt. Nach Beendigung der Styrolzugabe wurde 3 Stunden bei 135°C gerührt. Nach der Abtrennung des Katalysators und der destillativen Aufarbeitung erhielt man 96 Gramm (81% der Theorie) 6-(1'-Phenylethyl)-β-naphthol (Kp. 247—249°C/15 mbar). Der Rest bestand aus höher aralkylierten β-Naphtholen. Man beobachtete keine wesentlichen Mengen an Dinaphthylether und Styrolpolymeren.

Beispiel 4

(Vergleichsbeispiel)

In einem Rührreaktor wurde ein Gemisch aus 144 Gramm β-Naphthol und 5 Gramm Oxalsäure unter Rühren auf 130°C erhitzt und 104 Gramm Styrol innerhalb einer Stunde zugeführt. Nach Beendigung der Umsetzung wurde das Reaktionsgemisch im Vakuum destilliert. Man erhielt 213 Gramm (86% der Theorie) 1-(α-Phenylethyl)-β-naphthol (Kp. 172—176°C/0,2 mbar).

Beispiel 5

(Vergleichsbeispiel)

Ein Gemisch aus 144 Gramm β-Naphthol, 100 Milliliter Toluol und 15 Gramm Schwefelsäure (96%ig) wurde auf 110°C erhitzt und ein Gemisch aus 52 Gramm Styrol und 25 Milliliter Toluol innerhalb einer Stunde zugegeben. Nach 3 Stunden Erhitzen am Rückfluß wurde das Reaktionsgemisch auf übliche Weise aufgearbeitet. Neben 21 Gramm Naphtholsulfonsäure erhielt man 41 Gramm 6-(α-Phenylethyl)-2-naphthol (34% der Theorie) vom Kp. 244—248°C/15 mbar.

Beispiel 6

(Vergleichsbeispiel)

Ein Gemisch aus 144 Gramm β-Naphthol und 10 Gramm Amberlite IR-120 wurde auf 130°C erhitzt und 94 Gramm Styrol zugegeben. Anschließend wurde das Gemisch 15 Stunden bei 130°C gerührt. Entsprechend der gaschromatographischen Analyse enthielt das Reaktionsgemisch ein Gemisch aus 14% Naphthol, 63% 1-(1'-Phenylethyl)-2-naphthol, 10% 6-(1'-Phenylethyl)-2-naphthol, 3% 3- und 8-(1'-Phenylethylnaphthol) und 9% zweifach und höher alkylierte Naphthole.

**Patentanspruch**

Verfahren zur Herstellung von in 1-Stellung unsubstituierten, durch araliphatische Reste substituierten β-Naphtholen der Formel

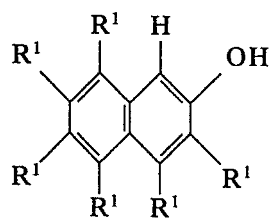

(I)

worin die einzelnen Reste $R^1$ gleich oder verschieden sein können, 1 bis 3 Reste $R^1$ jeweils den Rest

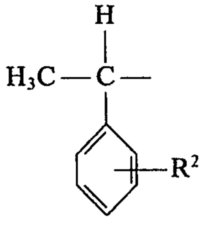

bedeuten, die übrigen Reste $R^1$ jeweils ein Wasserstoffatom oder einen Alkylrest mit 1 bis 8 Kohlenstoffatomen bezeichnen, $R^2$ für ein Wasserstoffatom oder einen Alkylrest mit 1 bis 8 Kohlenstoffatomen steht, durch Umsetzung von Naphtholen mit Styrolen in Gegenwart von aromatischen oder aliphatischen Sulfonsäuren, dadurch gekennzeichnet, daß man $\beta$-Naphthole der Formel

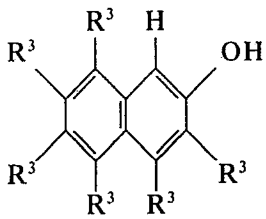

(II)

worin die einzelnen Reste $R^3$ gleich oder verschieden sein können und in den Stellungen, wo in der Formel I 1 bis 3 Reste $R^1$ den Rest

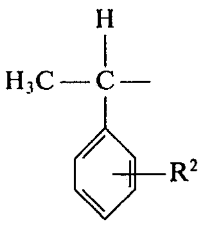

bedeuten, jeweils der entsprechende Rest $R^3$ ein Wasserstoffatom bezeichnet, die übrigen Reste $R^3$ dem Rest $R^1$ in der jeweiligen Stellung der Formel I entsprechend ein Wasserstoffatom oder den Alkylrest mit 1 bis 8 Kohlenstoffatomen bedeuten, mit Styrolen der Formel

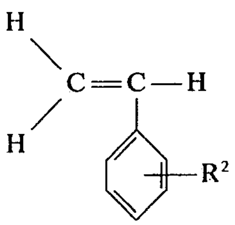

(III)

worin $R^2$ die vorgenannte Bedeutung besitzt, in Gegenwart von unter den Reaktionsbedingungen inerten, organischen Lösungsmitteln mit einem Siedepunkt über 130°C bei einer Temperatur von 100 bis 180°C umsetzt.

## Claim

A process for the preparation of $\beta$-naphthols which are substituted by araliphatic radicals and unsubstituted at the 1-position, and are of the formula

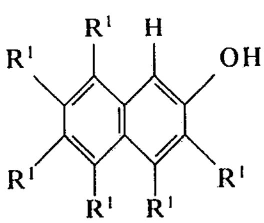

(I)

where the individual radicals $R^1$ may be identical or different, one to three radicals $R^1$ each denoting the radical

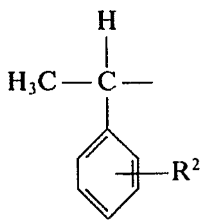

where $R^2$ is hydrogen or alkyl of 1 to 8 carbon atoms, and the remaining radicals $R^1$ each denoting hydrogen or alkyl of 1 to 8 carbon atoms, by reacting naphthols with styrenes in the presence of aromatic or aliphatic sulphonic acids, wherein $\beta$-naphthols of the formula

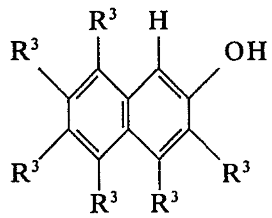

(II)

where the individual radicals $R^3$ may be identical or different, and at the positions where, in the formula I, one to three radicals $R^1$ each denote the radical

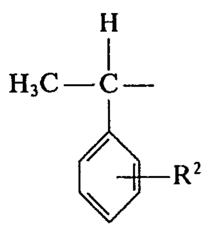

the corresponding radicals $R^3$ are each hydrogen, and each remaining radical $R^3$, being hydrogen or alkyl of 1 to 8 carbon atoms, has the same meaning as the radical $R^1$ at the corresponding position in the formula I, are reacted at from 100 to 180° C with styrenes of the formula

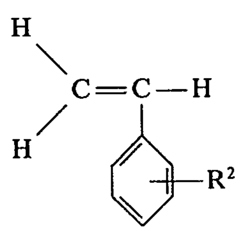

(III)

where $R^2$ has the above meanings, in the presence of an organic solvent which boils at above 130° C and is inert under the reaction conditions.

**Revendication**

Procédé de préparation de $\beta$-naphtols substitués par des groupes araliphatiques, mais non substitués en position 1, de la formule

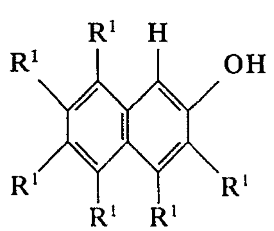

(I)

dans laquelle les divers substituants $R^1$ peuvent être identiques ou différents, un à trois substituants $R^1$ représentant un groupe

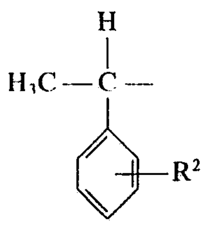

et les substituants $R^1$ restants un atome d'hydrogène ou un radical alkyle en $C_1$ à $C_8$ et $R^2$ désigne un atome d'hydrogène ou un radical alkyle en $C_1$ à $C_8$, par réaction de naphtols avec des styrènes en présence d'acides sulfoniques aliphatiques ou aromatiques, caractérisé en ce que l'on fait réagir un $\beta$-naphtol de la formule

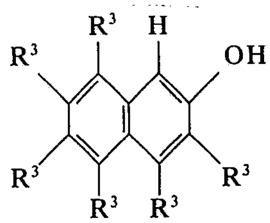

(II)

dans laquelle les divers substituants $R^3$ peuvent être identiques ou différents et désignent un atome d'hydrogène dans les positions où, dans la formule I, un à trois substituants $R^1$ représentent un groupe

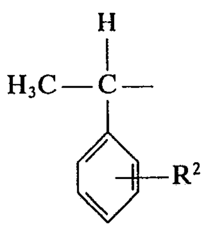

tandis que les substituants $R^3$ restants représentent un atome d'hydrogène ou un radical alkyle en $C_1$ à $C_8$ selon la signification du substituant $R^1$ dans la position correspondante dans la formule I, à une température de 100 à 180° C, dans un solvant organique d'un point d'ébullition supérieur à 130° C, inerte dans les conditions opératoires, avec un styrène de la formule

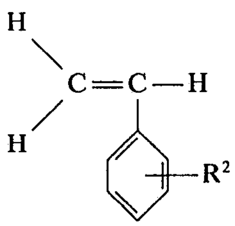

(III)

dans laquelle $R^2$ possède la signification définie.